# FASCICULE DE BREVET EUROPEEN

(11) **EP 2 237 865 B1**
(45) Date de publication et mention de la délivrance du brevet: **20.03.2019**
(21) Numéro de dépôt: 08870605.6
(22) Date de dépôt: 03.12.2008
(51) Int. Cl.: B01F 3/08, B01J 13/06, B01F 3/12, C08F 220/06, C08F 220/18, C08F 220/28

(54) **PROCEDE D'ENCAPSULATION DES HUILES EN MILIEU AQUEUX AVEC DES EMULSIONS POLYMERES DE TYPE HASE, PRODUITS OBTENUS ET LEURS UTILISATIONS**
VERFAHREN ZUR VERKAPSELUNG VON ÖLEN IN EINEM WÄSSRIGEN MEDIUM MIT HASE-POLYMEREMULSIONEN, ERHALTENE PRODUKTE UND VERWENDUNGEN DAVON
METHOD FOR ENCAPSULATING OILS IN AN AQUEOUS MEDIUM WITH HASE POLYMER EMULSIONS, PRODUCTS OBTAINED AND USES THEREOF

(30) Priorité: 20.12.2007 FR 0708888
(43) Date de publication de la demande: 13.10.2010
(73) Titulaire: COATEX, 69730 Genay (FR)
(72) Inventeur: MORO, Jean, F-01600 Saint Didier de Formans (FR); PLATEL, David, F-01800 Saint-Maurice de Gourdans (FR); SUAU, Jean-Marc, F-69480 Lucenay (FR); GUERRET, Olivier, F-69890 La Tour de Salvagny (FR)
(74) Mandataire: Balmefrezol, Ludovic Francis Pierre
(86) Numéro de dépôt international: PCT/IB2008/003393
(87) Numéro de publication internationale: WO 2009/090462

(56) Documents cités:
- EP-A- 0 870 785
- EP-A- 1 726 331
- WO-A-02/100374
- WO-A-03/062288
- WO-A-2006/016035
- WO-A-2007/101059
- US-A- 4 421 902
- US-A1- 2002 042 448
- US-A1- 2007 197 704

## Description

L'huile est un terme générique désignant des matières grasses qui ne se mélangent pas à l'eau. Les huiles sont des liquides gras, visqueux voire solides, d'origine animale, végétale, minérale ou synthétique. Parmi elles, on distingue :
- les huiles végétales et animales dites alimentaires, constituées par des lipides, et obtenues le plus souvent par pressage ;
- les huiles végétales dites essentielles qui correspondent aux liquides concentrés et hydrophobes des composés aromatiques volatiles d'une plante (mélanges de molécules variées, comprenant des terpènes comme des hydrates de carbone non aromatiques et des composés oxygénés à base d'alcools, d'aldéhydes ou de cétones), obtenues par distillation ou extraction chimique par solvants ;
- les huiles minérales qui sont des mélanges d'hydrocarbures, obtenues par raffinage ;
- les huiles de synthèse obtenues par synthèse chimique de molécules, ou hydrocraquage d'huiles minérales, et constituées de molécules très variées, à base carbonée, siliceuse, fluorée, etc...

Les huiles sont parfois mises en oeuvre dans des applications où l'eau est considérée comme un polluant ou un poison, mais elles sont aussi employées dans des formulations qui contiennent une phase aqueuse, certaines de ces formulations étant parfois même majoritairement constituées d'eau. Ces formulations aqueuses qui mettent en oeuvre des huiles de natures diverses sont, par exemple, des formulations cosmétiques, des peintures, des liants hydrauliques, des combustibles, des lubrifiants, des antimousse, des huiles de coupe et de trempe pour la métallurgie, des fertilisants, des formulations pharmaceutiques, agrochimiques, phytosanitaires, détergentes, alimentaires, ou des formulations du secteur de la fabrication du cuir, ou de l'enduction.

Initialement, on a mis au point différentes solutions en vue d'incorporer des huiles dans des formulations aqueuses tout en essayant de limiter les phénomènes indésirables (démixtion, séparation de phase, instabilité) inhérents à la présence d'une phase huileuse peu ou non dispersée dans l'eau. De tels phénomènes sont tout autant indésirables pour la formulation concernée d'un point de vue esthétique (apparition nette de la phase huileuse), que sur un plan rhéologique (altération des propriétés d'écoulement par exemple, liée à la présence de la phase huileuse non dispersée dans l'eau), ou encore au niveau de ses performances applicatives (le caractère inhomogène de la formulation, lié au mauvais état de dispersion de l'huile, peut conduire à une dégradation de ses performances finales). Ces solutions reposent notamment sur l'utilisation de solvants ou de mélanges de tensio-actifs, dont la fonction est de stabiliser la phase huileuse dans l'eau.

Outre les législations de plus en plus restrictives relatives à l'usage de solvants et de tensio-actifs, le développement d'une technologie plus récente a offert à l'homme du métier de nouvelles opportunités en vue de réaliser des formulations aqueuses contenant des huiles : celle de l'encapsulation. Cette technique permet d'isoler chimiquement ou physiquement une huile de la phase aqueuse dans laquelle elle est incorporée : on évite ainsi tout phénomène d'instabilité et on palie aux inconvénients mentionnés ci-dessus. On connaît à ce jour 2 grandes techniques d'encapsulation : la mise en oeuvre des β-cyclodextrines, et l'encapsulation à partir de polymères organiques.

La première catégorie met en oeuvre les β-cyclodextrines qui sont des molécules naturelles obtenues par dégradation enzymatique de l'amidon. Elles se présentent sous forme d'oligomères cycliques du glucose et sont caractérisées par la présence d'une cavité qui leur permet "d'accueillir une molécule hôte", de manière à former un complexe d'inclusion. A ce titre, le document JP 2001 354515 décrit l'encapsulation par une cyclodextrine d'une huile sous forme micronisée, pour un usage cosmétique.

La seconde catégorie est basée sur les procédés qui mettent en oeuvre des polymères organiques. Parmi ces derniers, on distingue les méthodes de coacervation : elles reposent sur l'enrobage d'une émulsion d'huiles par un film de polymères précipités à partir d'une solution colloïdale de ce polymère, ladite solution ayant été déstabilisée. Ce précipité, appelé coacervat, va s'adsorber sur les gouttelettes de l'émulsion d'huiles à enrober. A titre d'exemple, le document WO 00 / 48560 décrit la fabrication de microcapsules d'un mélange d'huiles et d'extrait de chlorophylle, par coacervation d'une solution aqueuse d'un alcool ayant de 2 à 4 atomes de carbone.
Une autre méthode d'encapsulation basée sur la mise en oeuvre de polymères est la polycondensation. Cette méthode repose sur la polycondensation de 2 monomères, l'un étant compatible avec le milieu d'encapsulation, l'autre étant compatible avec la substance à encapsuler, c'est-à-dire l'huile. Le document US 3 754 062 décrit l'encapsulation de produits gras par dissolution d'un polymère uréthanne et d'épichlorhydrine dans un liquide gras, dispersion du mélange résultant sous forme de gouttes dans une solution contenant une polyamine, et enfin polymérisation interfaciale du mélange. L'huile de ricin est citée à titre de produit encapsulable par cette technique.
Enfin, une troisième méthode d'encapsulation basée sur l'utilisation de polymères est celle qui met en oeuvre des polymères carboxylés à base d'un monomère qui est l'acide (méth)acrylique et d'un autre monomère qui est un ester acrylique (dans la littérature, ces polymères sont souvent désignés par le terme ASE qui signifie « émulsion alcali soluble »). Dans ce cas, le phénomène d'encapsulation est uniquement gouverné par la solubilité dudit polymère en fonction du pH. De tels polymères sont commercialement disponibles sous les appellations Eudragit™, Kollicoat™, Eastacryl 30D™. La méthode d'encapsulation consiste alors à mélanger ledit principe actif en solution aqueuse en présence dudit polymère, puis à sécher le mélange. Toutefois, comme le démontrent les exemples de la présente Demande, ces polymères de type ASE ne permettent pas d'encapsuler des huiles.
En parallèle, comme décrit dans US 4 421 902, un copolymère de type HASE peut être utilisé en tant qu'agent épaississant. Il permet notamment d'obtenir un concentrat eau-dans-huile par mélange avec un tensioactif, de la white spirit et de l'hydroxyde d'ammonium.

WO 03/062288 A1 décrit un procédé de fabrication d'une émulsion huile-dans-l'eau contenant une huile minérale, et caractérisé en ce qu'il comprend les étapes de 1) mélanger un polymère associatif constitué d'acide méthacrylique, d'acrylate d'éthyle en des monomères associatifs avec une huile minérale et de l'eau, dans des proportions telles que l'huile minérale représente 8% du poids total du mélange et 2) ajuster le pH du mélange à une valeur de 6.1 ou 6.5.

Poursuivant ses recherches pour encapsuler une huile, cette huile étant destinée à être introduite dans une formulation aqueuse, et contribuant ainsi à l'état de la technique à travers une solution alternative à celles proposées dans l'art antérieur, la Demanderesse a mis au point un procédé de fabrication d'une formulation aqueuse contenant au moins une huile, et caractérisé en ce qu'il comprend les étapes de :
a) mélanger au moins un polymère associatif constitué :
   - d'au moins un monomère qui est l'acide (méth)acrylique,
   - d'au moins un monomère qui est un ester (méth)acrylique,
   - et d'au moins un monomère hydrophobe associatif, avec au moins une huile et de l'eau, dans des proportions telles que l'huile représente au moins 4 % du poids total du mélange,
b) ajuster le pH du mélange obtenu à l'étape a) à une valeur supérieure à 6, préférentiellement 7, très préférentiellement 8,
c) précipiter le mélange obtenu après l'étape b) par ajustement du pH à une valeur inférieure à 6, en vue d'obtenir une dispersion dans l'eau de particules constituées dudit polymère et de ladite huile,
d) éventuellement isoler les particules constituées dudit polymère et de ladite huile obtenues après l'étape c) par élimination de l'eau.

Une des originalités du procédé selon l'invention est d'utiliser les polymères associatifs tels que décrits dans l'étape a) du procédé susmentionné. Lorsque ces polymères sont neutralisés à pH suffisamment élevé (supérieur à 6, préférentiellement 7, très préférentiellement 8), il y a création d'interactions associatives entre les groupements hydrophobes : ces interactions délimitent des domaines qui sont autant de cages de solvatation pour les molécules d'huile. Ces polymères sont souvent décrits dans la littérature à travers l'acronyme anglais HASE pour « émulsion alcali soluble modifiée hydrophobiquement ».

Un des mérites de la Demanderesse est d'avoir su identifier et utiliser le phénomène de structuration de l'eau via de tels polymères : on protège ainsi naturellement les huiles incorporées dans la formulation aqueuse. La Demanderesse souligne que ce mécanisme diffère du mode d'encapsulation par des polymères de type ASE, pour lesquels le phénomène d'encapsulation est uniquement gouverné par la solubilité dudit polymère en fonction du pH, comme déjà indiqué dans la présente Demande.

Une telle mise en oeuvre des polymères de type HASE est dans l'état de nos connaissances actuelles une utilisation nouvelle de ces objets largement décrits dans des applications pour la peinture (voir les documents FR 2 693 203, FR 2 872 815, FR 2 633 930), ou encore le secteur des bétons (voir la demande de brevet française portant le numéro de dépôt FR 07 00086 et non encore publiée à la date de dépôt de la présente demande).

En outre, la Demanderesse a aussi connaissance de la demande de brevet française non encore publiée à la date de dépôt de la présente demande et qui porte le numéro de dépôt FR 07 03890. Celle-ci décrit la mise en oeuvre de polymères de type HASE, en vue d'encapsuler des principes actifs odorants (selon l'essai n° 5 bis, seul essai révélant l'encapsulation d'un principe actif odorant qui est une huile de cananga). Cet essai mentionne que ladite huile est présente à raison de 3,33 % en poids par rapport au poids total de la formulation dans laquelle elle est incorporée : il s'agit d'un élément de différenciation indiscutable avec la présente invention, qui ne vise que des formulations contenant au moins 4 % en poids d'huile par rapport à leur poids total. C'est d'ailleurs un des avantages techniques offerts par la présente invention que de permettre d'encapsuler des molécules d'huile dans des quantités bien plus importantes : le pourcentage en poids d'huile peut être supérieur à 10 %, 40 % et même dans certains cas à 60 % du poids total de la formulation aqueuse considérée.

Par conséquent, après avoir réalisé les étapes a) et b) du procédé selon l'invention, on obtient un mélange dans lequel les molécules d'huile sont piégées dans des cages de solvatation.

Dans une première variante du procédé de l'invention, on met en oeuvre une étape c) d'acidification du mélange obtenu après l'étape b). Cette diminution du pH provoque l'effondrement de la structure du polymère : on obtient alors une dispersion dans l'eau de particules constituées du polymère et des molécules d'huile. Ces dernières molécules demeurent piégées.

Dans une deuxième variante, on peut mettre en oeuvre à la fois l'étape c) mais aussi une étape consécutive d), qui consiste en l'isolation des particules obtenues après l'étape c), par élimination de l'eau.

Aussi, un autre avantage du procédé est de délivrer une formulation aqueuse contenant un pourcentage en poids d'huile parfois très important (notamment supérieur à 60 % du poids total de ladite formulation) sous une forme qui piège les molécules d'huile, cette forme pouvant être triple :
- celle d'un liquide qui est une émulsion aqueuse, lorsque le produit est préparé en ne réalisant que les étapes a) de mélange puis b) d'ajustement du pH à une valeur supérieure à 6,
- celle d'un liquide qui est une dispersion dans l'eau de particules dudit polymère et de ladite huile, lorsque la préparation du produit met aussi en oeuvre l'étape de précipitation c) à un pH inférieur à 6,
- celle de particules solides constituées des molécules d'huile piégées dans les par les molécules de polymère, lorsqu'on a mis en oeuvre l'étape d) d'isolation du procédé selon l'invention.

La Demanderesse indique que l'unité d'invention est assurée entre ces 3 formes de réalisation décrites de part la mise en oeuvre dans chacune de ces formes du polymère de type HASE (copolymère de l'acide (méth)acrylique, d'un ester de ces acides, et d'un monomère hydrophobe associatif). Un autre mérite de la Demanderesse est qu'elle a su utiliser le comportement particulier de ce polymère de type HASE vis-à-vis du pH, en vue d'obtenir ces 3 modes de réalisationdécrits, qui apportent autant de souplesse et de liberté au formulateur.

Enfin, un dernier avantage de l'invention est qu'elle peut être mise en oeuvre pour piéger un très grand nombre de molécules d'huiles, quelle que soit leur origine. En effet, l'homme du métier a accès à une très vaste bibliothèque de monomères associatifs dans laquelle il peut puiser en vue d'identifier le monomère qui présentera la meilleure affinité possible vis-à-vis de la molécule d'huile qu'il désire piéger.

Un premier objet de l'invention est donc un procédé de fabrication d'une formulation aqueuse contenant au moins une huile, et caractérisé en ce qu'il comprend les étapes de :
a) mélanger au moins un polymère associatif constitué :
   - d'au moins un monomère qui est l'acide (méth)acrylique,
   - d'au moins un monomère qui est un ester (méth)acrylique,
   - et d'au moins un monomère hydrophobe associatif, avec au moins une huile et de l'eau, dans des proportions telles que l'huile représente au moins 4 % du poids total du mélange,
b) ajuster le pH du mélange obtenu à l'étape a) à une valeur supérieure à 6, préférentiellement 7, très préférentiellement 8,
c) précipiter le mélange obtenu après l'étape b) par ajustement du pH à une valeur inférieure à 6, en vue d'obtenir une dispersion dans l'eau de particules constituées dudit polymère et de ladite huile,
d) éventuellement isoler les particules constituées dudit polymère et de ladite huile obtenues après l'étape c) par élimination de l'eau.

Dans une première variante non revendiquée, le procédé met en oeuvre exclusivement les étapes a) et b).

Dans une deuxième variante revendiquée, le procédé selon l'invention met en oeuvre exclusivement les étapes a), b) et c).

Dans une troisième variante revendiquée, le procédé selon l'invention met en oeuvre exclusivement les étapes a), b), c), et d).

Le procédé selon l'invention est aussi caractérisé en ce qu'on met en oeuvre, dans l'étape a), de 0,1 % à 20 %, préférentiellement de 0,1 % à 10 %, très préférentiellement de 0,1 % à 5 % en poids sec dudit polymère associatif, par rapport au poids total du mélange obtenu après l'étape a).

Le procédé selon l'invention est aussi caractérisé en ce qu'on met en oeuvre, pendant l'étape a), au moins 4 %, préférentiellement au moins 10 %, très préférentiellement au moins 40 %, de façon extrêmement préférentielle au moins 60 % en poids d'au moins une huile, et au plus 70 % en poids d'au moins une huile, par rapport au poids total du mélange obtenu après l'étape a).

Le procédé selon l'invention est aussi caractérisé en ce que le pH du mélange, au cours de l'étape b), est ajusté au moyen d'une base organique ou minérale. Pratiquement, les constituants (huile, eau, polymère associatif, ainsi que la base minérale ou organique) sont introduits sous agitation dans un réacteur ; l'ordre d'introduction sera choisi par l'homme du métier, notamment en fonction de la solubilité dans l'eau de l'huile à encapsuler.

Le procédé selon l'invention est aussi caractérisé en ce qu'on met en oeuvre un acide moyennement fort ou fort au cours de l'étape c).

Le procédé selon l'invention est aussi caractérisé en ce que le monomère qui est un ester (méth)acrylique est choisi préférentiellement parmi l'acrylate d'éthyle, l'acrylate de butyle, le méthacrylate de méthyle et leurs mélanges.

Le procédé selon l'invention est aussi caractérisé en ce que ledit monomère hydrophobe associatif possède la formule générale (I) : 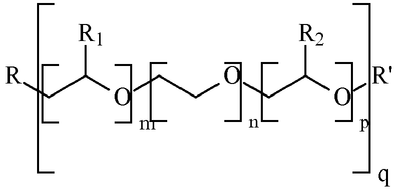 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

Le procédé selon l'invention est enfin caractérisé en ce que l'eau est éliminée par évaporation ou centrifugation au cours de l'étape d). Toutefois, l'homme du métier pourra mettre en oeuvre toute autre technique visant à éliminer l'eau du mélange obtenu après les étapes a) et b).

Le procédé selon l'invention est aussi caractérisé en ce que l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

Il est décrit la formulation aqueuse contenant au moins une huile, et obtenue par la mise en oeuvre des étapes a) et b) du procédé précédemment décrit.

Cette formulation aqueuse, contenant au moins une huile, est caractérisée en ce qu'elle contient de l'eau, au moins une huile et au moins un polymère hydrophobe associatif constitué :
- d'au moins un monomère qui est l'acide (méth)acrylique,
- d'au moins un monomère qui est un ester (méth)acrylique,
- et d'au moins un monomère hydrophobe associatif,
en ce qu'elle possède un pH supérieur à 6, préférentiellement 7, très préférentiellement 8, et en ce qu'elle contient au moins 4 % en poids d'huile par rapport à son poids total.

Cette formulation aqueuse est aussi caractérisée en ce qu'elle contient de 0,1 % à 20 %, préférentiellement de 0,1 % à 10 %, très préférentiellement de 0,1 % à 5 % en poids sec d'au moins un polymère hydrophobe associatif, par rapport à son poids total.
Cette formulation aqueuse est aussi caractérisée en ce qu'elle contient au moins 4 %, préférentiellement au moins 10 %, très préférentiellement au moins 40 %, de façon extrêmement préférentielle au moins 60 % en poids d'au moins une huile, et au plus 70 % en poids d'au moins une huile, par rapport à son poids total.

Cette formulation aqueuse est aussi caractérisée en ce que le monomère qui est un ester (méth)acrylique est choisi préférentiellement parmi l'acrylate d'éthyle, l'acrylate de butyle, le méthacrylate de méthyle et leurs mélanges.

Cette formulation aqueuse est aussi caractérisée en ce que ledit monomère hydrophobe associatif possède la formule générale (I) : 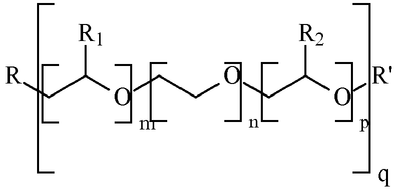 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

Cette formulation aqueuse est aussi caractérisée en ce que l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

Un autre objet de l'invention consiste en la formulation constituée de particules solides en dispersion dans l'eau, et obtenue par mise en oeuvre de l'étape de précipitation c) du procédé auparavant décrit.

Cette dispersion de particules dans l'eau est caractérisée en ce que lesdites particules contiennent au moins une huile et au moins un polymère hydrophobe associatif constitué :
- d'au moins un monomère qui est l'acide (méth)acrylique,
- d'au moins un monomère qui est un ester (méth)acrylique,
- et d'au moins un monomère hydrophobe associatif,
en ce qu'elle possède un pH inférieur à 6,
et en ce qu'elle contient au moins 4 % en poids d'huile par rapport à son poids total.

Cette dispersion de particules dans l'eau est aussi caractérisée en ce qu'elle contient de 0,1 % à 20 %, préférentiellement de 0,1 % à 10 %, très préférentiellement de 0,1 % à 5 % en poids sec d'au moins un polymère hydrophobe associatif, par rapport à son poids total.

Cette dispersion de particules dans l'eau est aussi caractérisée en ce qu'elle contient au moins 4 %, préférentiellement au moins 10 %, très préférentiellement au moins 40 %, de façon extrêmement préférentielle au moins 60 % en poids d'au moins une huile, et au plus 70 % en poids d'au moins une huile, par rapport à son poids total.

Cette dispersion de particules dans l'eau est aussi caractérisée en ce que le monomère qui est un ester (méth)acrylique est choisi préférentiellement parmi l'acrylate d'éthyle, l'acrylate de butyle, le méthacrylate de méthyle et leurs mélanges.

Cette dispersion de particules dans l'eau est aussi caractérisée en ce que ledit monomère hydrophobe associatif possède la formule générale (I) : 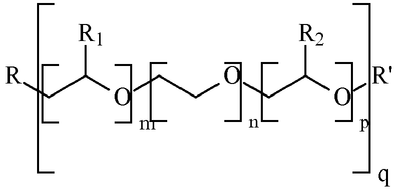 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

Cette dispersion de particules dans l'eau est aussi caractérisée en ce que l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

Il est également décrit la formulation constituée des particules solides obtenues par mise en oeuvre de l'étape d'isolation d) du procédé auparavant décrit.

Ces particules solides sont caractérisées en ce qu'elles contiennent au moins une huile et au moins un polymère hydrophobe associatif constitué :
- d'au moins un monomère qui est l'acide (méth)acrylique,
- d'au moins un monomère qui est un ester (méth)acrylique,
- et d'au moins un monomère hydrophobe associatif.

Ces particules solides sont aussi caractérisées en ce que ledit monomère hydrophobe associatif possède la formule générale (I) : 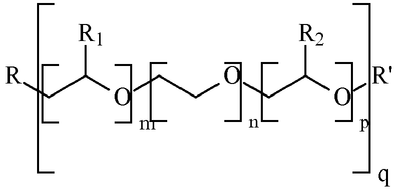 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

Ces particules solides sont aussi caractérisées en ce que l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

Un dernier objet de l'invention est l'utilisation des formulations aqueuses contenant au moins une huile, des dispersions aqueuses de particules solides contenant au moins une huile, dans les domaines de la cosmétique, la peinture, la construction, les combustibles, les lubrifiants, les antimousse, la métallurgie, les fertilisants, la pharmacie, l'agrochimique, le phytosanitaire, la détergence, l'alimentation, le cuir, l'enduction.

### EXEMPLES

Les exemples 1 et 2 illustrent respectivement la fabrication de monomères pouvant être mis en oeuvre selon l'invention, de même que la fabrication (à partir de ces monomères) de polymères associatifs hydrosolubles pouvant être mis en oeuvre selon l'invention.

### Exemple 1 : Synthèse de monomères mis en oeuvre selon l'invention

### Protocole a : Synthèse de monomère méthacrylique

Dans un réacteur de 1 litre, on pèse :
- 400 grammes de l'alcool béhénique condensé avec 25 moles d'oxyde d'éthylène fondu,
- 0,0994 gramme d'alloocimène,
- 43,75 grammes d'anhydride méthacrylique.
On chauffe le mélange à 82°C ± 2°C et on poursuit la cuisson pendant 3 heures à cette température. Le macromonomère obtenu est alors dilué avec 396 g d'acide méthacrylique afin d'obtenir une solution liquide à température ambiante.

### Protocole b : Synthèse de monomère uréthanne

Dans une première étape, on réalise un précondensat en pesant dans un erlen :
- 13,726 grammes de toluène di isocyanate,
- 36,1 grammes d'acrylate d'éthyle,
- 0,077 gramme d'alloocimène,
- 0,198 grammes de dilaurate de dibuthyle d'étain.
On pèse ensuite dans une ampoule de coulée 10,257 grammes de méthacrylate d'éthylène glycol et 10 grammes d'acrylate d'éthyle. On coule le contenu de cette ampoule dans l'erlen en 20 minutes à une température inférieure à 35°C, et on laisse réagir 30 minutes.

Dans une deuxième étape, on réalise la condensation en pesant 132 grammes de tri styryl phénol condensé avec 25 moles d'oxyde d'éthylène dans un réacteur de 1000 ml que l'on maintiendra fondu à 65°C. On coule alors le précondensat en 20 minutes à 65°C sur cet alcool puis on cuit 2 heures à 65°C. Enfin, on dilue le mélange avec 15,5 grammes d'acrylate d'éthyle et 84,6 grammes d'eau bi permutée afin d'obtenir un liquide à température ambiante.

### Protocole c : Synthèse de monomère hémimaléate

Dans un réacteur de 1 litre on pèse :
- 400 grammes de l'alcool ramifié de 32 atomes de carbone condensé avec 25 moles d'oxyde d'éthylène fondu,
- 0,0994 gramme d'alloocimène,
- 25,3 grammes d'anhydride maléique.
On chauffe le mélange à 82°C ± 2°C et on poursuit la cuisson pendant 3 heures. Le macromonomère alors obtenu est dilué avec 425 g d'acide méthacrylique afin d'obtenir une solution liquide à température ambiante.

### Exemple 2 : Synthèse de polymères hydrosolubles associatifs

### Protocole A

Dans un réacteur de 1 litre, on pèse 280 grammes d'eau bi permutée et 3,89 grammes de dodecyl sulfate de sodium. On chauffe en pied de cuve à 82°C ± 2°C.
Pendant ce temps, on prépare une pré émulsion, en pesant dans un bécher :
- 112,4 grammes d'eau bi permutée,
- 2,1 grammes de dodecyl sulfate de sodium,
- 80,6 grammes d'acide méthacrylique,
- 146,1 grammes d'acrylate d'éthyle,
- 55,6 grammes d'une solution de macromonomère telle que décrite dans le protocole a).
On pèse ensuite 0,85 gramme de persulfate d'ammonium dilués dans 10 grammes d'eau bi permutée pour le premier catalyseur, et 0,085 gramme de métabisulfite de sodium dilués dans 10 grammes d'eau bi permutée pour le second catalyseur. Lorsque le pied de cuve est à température, on ajoute les 2 catalyseurs et on effectue la polymérisation pendant 2 heures à 76°C ± 2°C, avec ajout en parallèle de la pré émulsion. On rince la pompe avec 20 grammes d'eau bi permutée et on cuit 1 heure à 76°C ± 2°C. On refroidit enfin à température ambiante et on filtre le polymère ainsi obtenu.

### Protocole B

Dans un réacteur de 1 litre on pèse 280 grammes d'eau bi permutée et 3,89 grammes de dodecyl sulfate de sodium. On chauffe en pied de cuve à 82°C ± 2°C.
Pendant ce temps, on prépare une pré émulsion, en pesant dans un bécher :
- 334 grammes d'eau bi permutée,
- 3,89 grammes de dodecyl sulfate de sodium,
- 0,92 gramme de dodecyl mercaptan,
- 80,6 grammes d'acide méthacrylique,
- 160,55 grammes d'acrylate d'éthyle,
- 60,4 grammes de la solution de méthacryluréthanne décrite dans le protocole b).
On pèse ensuite 0,33 gramme de persulfate d'ammonium dilués dans 10 grammes d'eau bi permutée pour le premier catalyseur, et 0,28 gramme de métabisulfite de sodium dilués dans 10 grammes d'eau bi permutée pour le second catalyseur. Lorsque le pied de cuve est à température, on ajoute les 2 catalyseurs et on effectue la polymérisation pendant 2 heures à 84°C ± 2°C, avec ajout en parallèle de la pré émulsion. On rince la pompe avec 20 grammes d'eau bi permutée et on cuit 1 heure à 84°C ± 2°C. On refroidit enfin à température ambiante et on filtre.

### Protocole C

Ce protocole est identique au protocole B, à la différence près qu'on supprime ici le dodecyl mercaptan dans la première étape de pesée.

### Protocole D

Ce protocole est identique au protocole A, à la différence près qu'on introduit 0,9 gramme de dodecyl mercaptan dans l'étape de pesée initiale dans le bêcher.

### Exemple 3

Cet exemple illustre le procédé selon l'invention, dans lequel on met en oeuvre les étapes a) de mélange, b) d'augmentation du pH, et c) de diminution du pH. Il démontre notamment que l'encapsulation a lieu lorsque se développent les interactions associatives des groupes hydrophobes du polymère selon l'invention, ces interactions étant commandées par les variations spécifiques du pH que la Demanderesse a su utiliser pour encapsuler une huile. Cet exemple illustre la mise en oeuvre de différents polymères pour encapsuler une huile qui est une huile de tournesol.

### Essai n° 1

Cet essai illustre un essai hors invention.
On commence par peser 16,5 grammes d'un polymère constitué de 36,9 % en poids d'acide méthacrylique et 63,1 % en poids d'acrylate d'éthyle, sous forme d'une dispersion contenant 30 % en poids sec dudit polymère, 182 grammes d'eau et 150 grammes d'une huile de tournesol alimentaire distribuée par la société ORGELEC™. Le pH est alors égal à 2.
Ce polymère est un polymère bien connu de l'homme du métier sous le vocable d'épaississant de type ASE dont on a déjà décrit le mode d'encapsulation dans le cas des principes actifs. Or, on constate ici, après le simple mélange précédemment décrit, qu'on obtient 2 phases : l'une huileuse, et l'autre aqueuse contenant le polymère.
On a alors augmenté progressivement le pH par des ajouts successifs de soude, par paliers de 0,5 unité pH, jusqu'à une valeur finale égale à 9.
Or, après stabilisation du pH à chacune de ces valeurs-paliers, on continue à observer un système diphasique.
Ensuite, à partir de la valeur de pH égale à 9, on a diminué celui-ci par paliers successifs de 0,5 unités pH par ajouts successifs d'acide phosphorique, jusqu'à une valeur finale égale à 5.
Or, après stabilisation du pH à chacune de ces valeurs-paliers, on continue à observer un système diphasique.
Ceci démontre donc bien que les polymères de type ASE ne permettent pas d'encapsuler des huiles.

### Essai n° 2 à 12

Ces essais illustrent l'invention.
On commence par peser 16,5 grammes de polymère sous forme d'une dispersion contenant 30 % en poids sec dudit polymère, 182 grammes d'eau et 150 grammes d'une huile de tournesol alimentaire distribuée par la société ORGELEC™.
Selon l'étape a), on réalise le mélange.
Selon l'étape b), on augmente le pH à une valeur égale à 8 par ajout de soude.
Selon l'étape c), on diminue ensuite le pH à une valeur égale à 5,9.

La composition des polymères est dans le tableau 1.

On obtient une dispersion dans l'eau de particules constituées d'huile et du polymère : les polymères mis en oeuvre permettent donc d'encapsuler efficacement l'huile de tournesol.

La taille de ces dispersions a été déterminée par diffusion dynamique de la lumière à l'aide d'un Zétasizer™ nano S90 commercialisé par la société MALVERN™.

On a également déterminé la stabilité au stockage de ces dispersions, par simple observation visuelle. On considère qu'il n'y a plus stabilité lorsqu'on perd l'homogénéité de la dispersion (phénomènes de sédimentation ou de crémage en surface). Ces résultats sont dans le tableau 2.

**Tableau 1**

| Essai n° | x | Y | z | R₁ | R₂ | R | R' | m | n | p | q |
|---|---|---|---|---|---|---|---|---|---|---|---|
| 2 | 37,4 | 54,3 | 8,3 | - | - | méthacrylate | alkyle ramifié avec 16 atomes de carbone | 0 | 25 | 0 | 1 |
| 3 | 36,7 | 53,1 | 10,2 | - | - | méthacrylate | alkyle linéaire avec 22 atomes de carbone | 0 | 25 | 0 | 1 |
| 4 | 36,7 | 53,1 | 10,2 | CH₃ | - | méthacrylate | alkyle linéaire avec 22 atomes de carbone | 5 | 25 | 0 | 1 |
| 5 | 36,0 | 53,7 | 10,3 | - | - | méthacrylate | alkyle ramifié avec 20 atomes de carbone | 0 | 30 | 0 | 1 |
| 6 | 36,0 | 53,7 | 10,3 | - | - | méthacrylate | alkyle linéaire avec 18 atomes de carbone | 0 | 20 | 0 | 1 |
| 7 | 37,4 | 54,3 | 8,3 | - | - | méthacrylate | TSP | 0 | 40 | 0 | 1 |
| 8 | 40,3 | 54,7 | 5,0 | - | - | Méthacryl uréthanne | nonylphénol | 0 | 50 | 0 | 1 |
| 9 | 34,6 | 57,7 | 7,7 | - | - | méthacrylate | alkyle ramifié avec 28 atomes de carbone | 0 | 25 | 0 | 1 |
| 10 | 36,5 | 54,1 | 9,4 | - | - | méthacryl uréthanne | alkyle linéaire avec 12 atomes de carbone | 0 | 25 | 0 | 1 |
| 11 | 37,3 | 54,8 | 7,9 | - | - | méthacrylate | alkyle ramifié avec 32 atomes de carbone | 0 | 25 | 0 | 1 |
| 12 | 37,3 | 54,8 | 7,9 | - | - | méthacrylate | alkyle ramifié avec 32 atomes de carbone | 0 | 40 | 0 | 1 |

| | | | | | | | | | | | |
|---|---|---|---|---|---|---|---|---|---|---|---|
| TSP : tristyrylphénol (30 atomes de carbone) | | | | | | | | | | | |

Avec les notations définies dans la formule (I), x y et z désignant respectivement les ratio massiques d'acide (méth)acrylique, d'ester (méth)acrylique, et du monomère de formule (I)

**Tableau 2**

| Essai n° | Diamètre (nm) | Stockage (jours) |
|---|---|---|
| 2 | 4030 | 40 |
| 3 | 4680 | > 92 |
| 4 | 4300 | 85 |
| 5 | 1950 | > 95 |
| 6 | 3780 | 65 |
| 7 | 3530 | 65 |
| 8 | 460 | 27 |
| 9 | 3280 | 65 |
| 10 | 460 | 7 |
| 11 | 3470 | 12 |
| 12 | 3850 | 18 |

On constate notamment ici l'efficacité accrue des polymères mis en oeuvre pour les essais n° 3, 4 et 5.

### Exemple 4

Cet exemple illustre le procédé selon l'invention, dans lequel on met en oeuvre les étapes a) de mélange, b) d'augmentation du pH, et c) de diminution du pH. On travaille ici avec de l'huile de tournesol et avec le polymère testé selon l'essai n° 2.

### Essai n° 13

On commence par peser 16,5 grammes de polymère sous forme d'une dispersion contenant 30 % en poids sec dudit polymère, 162 grammes d'eau et 200 grammes d'une huile de tournesol alimentaire distribuée par la société ORGELEC™.
Selon l'étape a), on réalise le mélange.
Selon l'étape b), on augmente le pH à une valeur égale à 8 par ajout de soude.
Selon l'étape c), on diminue ensuite le pH à une valeur égale à 5,8.

### Essai n° 14

On commence par peser 16,5 grammes de polymère sous forme d'une dispersion contenant 30 % en poids sec dudit polymère, 162 grammes d'eau et 250 grammes d'une huile de tournesol alimentaire distribuée par la société ORGELEC™.
Selon l'étape a), on réalise le mélange.
Selon l'étape b), on augmente le pH à une valeur égale à 8,1 par ajout de soude.
Selon l'étape c), on diminue ensuite le pH à une valeur égale à 5,8.

### Essai n° 15

On commence par peser 16,5 grammes de polymère sous forme d'une dispersion contenant 30 % en poids sec dudit polymère, 162 grammes d'eau et 300 grammes d'une huile de tournesol alimentaire distribuée par la société ORGELEC™.
Selon l'étape a), on réalise le mélange.
Selon l'étape b), on augmente le pH à une valeur égale à 8,1 par ajout de soude.
Selon l'étape c), on diminue ensuite le pH à une valeur égale à 5,8.

### Essai n° 16

On commence par peser 16,5 grammes de polymère sous forme d'une dispersion contenant 30 % en poids sec dudit polymère, 162 grammes d'eau et 350 grammes d'une huile de tournesol alimentaire distribuée par la société ORGELEC™.
Selon l'étape a), on réalise le mélange.
Selon l'étape b), on augmente le pH à une valeur égale à 8,1 par ajout de soude.
Selon l'étape c), on diminue ensuite le pH à une valeur égale à 5,8.

On obtient une dispersion dans l'eau de particules constituées d'huile et du polymère : le polymère mis en oeuvre permet donc d'encapsuler efficacement les huiles. La taille de ces dispersions a été déterminée par diffusion dynamique de la lumière à l'aide d'un Zétasizer™ nano S90 commercialisé par la société MALVERN™. Les résultats sont dans le tableau 3.

**Tableau 3**

| **Essai n°** | **Diamètre (nm)** |
|---|---|
| 13 | 4240 |
| 14 | 4360 |
| 15 | 4360 |
| 16 | 4620 |

### Exemple 5

Cet exemple illustre le procédé selon l'invention, dans lequel on met en oeuvre les étapes a) de mélange, b) d'augmentation du pH, et c) de diminution du pH.
On travaille ici avec différentes huiles et différents polymères.

Les essais n° 17 à 19 mettent en oeuvre un polymère constitué de 26,3 % en poids d'acrylate d'éthyle, 26,3 % de méthacrylate de méthyle, 38,2 d'acide méthacrylique et 9,2 % d'un monomère de formule (I) dans laquelle :
- R désigne le groupe méthacrylate
- m=p=0 n=25 q=1
- R' désigne le radical alkyle linéaire avec 22 atomes de carbone.

### Essai n° 17

On commence par peser 33 grammes de polymère sous forme d'une dispersion contenant 30 % en poids sec dudit polymère, 150 grammes d'eau et 41,6 grammes de citronellal. Selon l'étape a), on réalise le mélange.
Selon l'étape b), on augmente le pH à une valeur égale à 8,9 par ajout de soude.
Selon l'étape c), on diminue ensuite le pH à une valeur égale à 5,8.

### Essai n° 18

On commence par peser 33 grammes de polymère sous forme d'une dispersion contenant 30 % en poids sec dudit polymère, 150 grammes d'eau et 26,2 grammes de citronellal.
Selon l'étape a), on réalise le mélange.
Selon l'étape b), on augmente le pH à une valeur égale à 8,2 par ajout de soude.
Selon l'étape c), on diminue ensuite le pH à une valeur égale à 5,7.

### Essai n° 19

On commence par peser 33 grammes de polymère sous forme d'une dispersion contenant 30 % en poids sec dudit polymère, 150 grammes d'eau et 12,4 grammes de citronellal.
Selon l'étape a), on réalise le mélange.
Selon l'étape b), on augmente le pH à une valeur égale à 8,2 par ajout de soude.
Selon l'étape c), on diminue ensuite le pH à une valeur égale à 5,9.

### Essai n° 20

Cet essai met en oeuvre le polymère testé dans l'essai n° 2.
On commence par peser 16,5 grammes de polymère sous forme d'une dispersion contenant 30 % en poids sec dudit polymère, 182 grammes d'eau et 150 grammes d'une huile silicone.
Selon l'étape a), on réalise le mélange.
Selon l'étape b), on augmente le pH à une valeur égale à 8,2 par ajout de soude.
Selon l'étape c), on diminue ensuite le pH à une valeur égale à 5,9.

### Essai n° 21

Cet essai met en oeuvre le polymère testé dans l'essai n° 11.
On commence par peser 16,5 grammes de polymère sous forme d'une dispersion contenant 30 % en poids sec dudit polymère, 182 grammes d'eau et 24 grammes d'alcool béhénique.
Selon l'étape a), on réalise le mélange, en chauffant à 85 °C.
Selon l'étape b), on augmente le pH à une valeur égale à 8,1 par ajout de soude.
Selon l'étape c), on diminue ensuite le pH à une valeur égale à 5,9.

### Essai n° 22

Cet essai met en oeuvre le polymère testé dans les essais n° 17 à 19.
On commence par peser 16,5 grammes de polymère sous forme d'une dispersion contenant 30 % en poids sec dudit polymère, 182 grammes d'eau et 48 grammes d'alcool béhénique.
Selon l'étape a), on réalise le mélange, en chauffant à 85 °C.
Selon l'étape b), on augmente le pH à une valeur égale à 8,1 par ajout de soude.
Selon l'étape c), on diminue ensuite le pH à une valeur égale à 5,9.

### Essai n° 23

Cet essai met en oeuvre le polymère testé dans l'essai n° 10.
On commence par peser 4,95 grammes de polymère sous forme d'une dispersion contenant 30 % en poids sec dudit polymère, 45,5 grammes d'eau et 37,5 grammes de tetradodécane.
Selon l'étape a), on réalise le mélange, en chauffant à 85 °C.
Selon l'étape b), on augmente le pH à une valeur égale à 8,1 par ajout de soude.
Selon l'étape c), on diminue ensuite le pH à une valeur égale à 5,9.

On obtient une dispersion dans l'eau de particules constituées d'huile et du polymère : le polymère mis en oeuvre permet donc d'encapsuler efficacement les huiles. La taille de ces dispersions a été déterminée par diffusion dynamique de la lumière à l'aide d'un Zétasizer™ nano S90 commercialisé par la société MALVERN™. Les résultats sont dans le tableau 4.

**Tableau 4**

| **Essai n°** | **Diamètre (nm)** |
|---|---|
| 17 | 1800 |
| 18 | 650 |
| 19 | 520 |
| 20 | 2060 |
| 21 | 4400 |
| 22 | 5200 |
| 23 | 2200 |

## Revendications

1. Procédé de fabrication d'une formulation aqueuse ou éventuellement de particules solides contenant au moins une huile, et **caractérisé en ce qu'**il comprend les étapes de :
a) mélanger au moins un polymère associatif constitué :
- d'au moins un monomère qui est l'acide (méth)acrylique,
- d'au moins un monomère qui est un ester (méth)acrylique,
- et d'au moins un monomère hydrophobe associatif,
avec au moins une huile et de l'eau, dans des proportions telles que l'huile représente au moins 4 % du poids total du mélange,
b) ajuster le pH du mélange obtenu à l'étape a) à une valeur supérieure à 6, préférentiellement 7, très préférentiellement 8, et obtention d'une émulsion aqueuse
c) précipiter le mélange obtenu après l'étape b) par ajustement du pH à une valeur inférieure à 6, en vue d'obtenir une dispersion dans l'eau de particules constituées dudit polymère et de ladite huile,
d) éventuellement isoler les particules constituées dudit polymère et de ladite huile obtenues après l'étape c) par élimination de l'eau.

2. Procédé selon la revendication 1, **caractérisé en ce qu'**il met en oeuvre exclusivement les étapes a), b), c) et d).

3. Procédé selon l'une des revendications 1 à 2, **caractérisé en ce qu'**on met en oeuvre, dans l'étape a), de 0,1 % à 20 %, préférentiellement de 0,1 % à 10 %, très préférentiellement de 0,1 % à 5 % en poids sec dudit polymère associatif, par rapport au poids total du mélange obtenu après l'étape a).

4. Procédé selon l'une des revendications 1 à 3, **caractérisé en ce qu'**on met en oeuvre, pendant l'étape a), au moins 4 %, préférentiellement au moins 10 %, très préférentiellement au moins 40 %, de façon extrêmement préférentielle au moins 60 % en poids d'au moins une huile, et au plus 70 % en poids d'au moins une huile, par rapport au poids total du mélange obtenu après l'étape a).

5. Procédé selon l'une des revendications 1 à 4, **caractérisé en ce que** le pH du mélange, au cours de l'étape b), est ajusté au moyen d'une base organique ou minérale.

6. Procédé selon l'une des revendications 1 à 5, **caractérisé en ce qu'**on met en oeuvre un acide moyennement fort ou fort au cours de l'étape c).

7. Procédé selon l'une des revendications 1 à 6, **caractérisé en ce que** le monomère qui est un ester (méth)acrylique est choisi préférentiellement parmi l'acrylate d'éthyle, l'acrylate de butyle, le méthacrylate de méthyle et leurs mélanges.

8. Procédé selon l'une des revendications 1 à 7, **caractérisé en ce que** ledit monomère hydrophobe associatif possède la formule générale (I) : 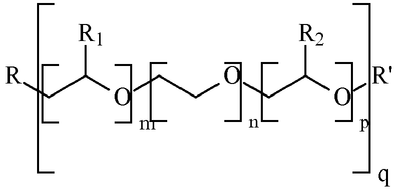 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

9. Procédé selon l'une des revendications 1 à 8, **caractérisé en ce que** l'eau est éliminée par évaporation ou centrifugation au cours de l'étape d).

10. Procédé selon l'une des revendications 1 à 9, **caractérisé en ce que** l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

11. Dispersion de particules dans l'eau obtenue suite à l'étape c) du procédé selon l'une quelconque des revendications 1 et 3 à 10, **caractérisée en ce que** lesdites particules contiennent au moins une huile et au moins un polymère hydrophobe associatif constitué :
- d'au moins un monomère qui est l'acide (méth)acrylique,
- d'au moins un monomère qui est un ester (méth)acrylique,
- et d'au moins un monomère hydrophobe associatif,
**en ce qu'**elle possède un pH inférieur à 6,
et **en ce qu'**elle contient au moins 4 % en poids d'huile par rapport à son poids total.

12. Dispersion de particules dans l'eau selon la revendication 11, **caractérisée en ce qu'**elle contient de 0,1 % à 20 %, préférentiellement de 0,1 % à 10 %, très préférentiellement de 0,1 % à 5 % en poids sec d'au moins un polymère hydrophobe associatif, par rapport à son poids total.

13. Dispersion de particules dans l'eau selon l'une des revendications 11 ou 12, **caractérisée en ce qu'**elle contient au moins 4 %, préférentiellement au moins 10 %, très préférentiellement au moins 40 %, de façon extrêmement préférentielle au moins 60 % en poids d'au moins une huile, et au plus 70 % en poids d'au moins une huile, par rapport à son poids total.

14. Dispersion de particules dans l'eau selon l'une des revendications 11 à 13, **caractérisée en ce que** le monomère qui est un ester (méth)acrylique est choisi préférentiellement parmi l'acrylate d'éthyle, l'acrylate de butyle, le méthacrylate de méthyle et leurs mélanges.

15. Dispersion de particules dans l'eau selon l'une des revendications 11 à 14, **caractérisée en ce que** ledit monomère hydrophobe associatif possède la formule générale (I) : 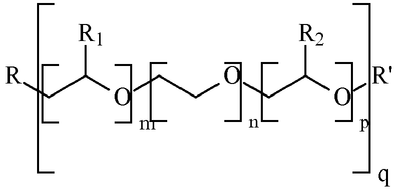 où :
- m, n, p et q sont des entiers et m, n, p sont inférieurs à 150, q est supérieur à 0 et au moins un entier parmi m, n et p est non nul
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone, préférentiellement au moins 16 et au plus 24 atomes de carbone, très préférentiellement au moins 18 et au plus 22 atomes de carbone.

16. Dispersion de particules dans l'eau selon l'une des revendications 11 à 15, **caractérisées en ce que** l'huile est choisie parmi les huiles de microalgues, de Pongamia pinnata, de Jatropha, de palme, de tournesol, de colza, d'amande, d'arachide, de coprah, de lin, de maïs, d'olive, de pépins de raisin, de ricin, de sésame, de moutarde, de noix, de soja, de baleine, de cachalot, de foie de morue, pied de boeuf, le suif ou graisse de boeuf, le saindoux ou gras de porc, les huiles de bourrache, de jojoba, de macadamia, de millepertuis, de noisettes, de rose musquée, de noyaux d'abricots, de germes de blé, d'onagre, les graisses de canard, de poulet, les acides oléique, palmitique, linoléique, stéarique, les huiles moteur.

17. Utilisation des dispersions aqueuses selon l'une des revendications 11 à 16, dans les domaines de la cosmétique, la peintures, la construction, des combustibles, des lubrifiants, des antimousse, la métallurgie, les fertilisants, la pharmacie, l'agrochimique, le phytosanitaire, la détergence, l'alimentation, le cuir, l'enduction.

## Patentansprüche

1. Verfahren zur Herstellung einer wässrigen Formulierung oder gegebenenfalls einer Formulierung von festen Teilchen, die mindestens ein Öl enthält, **dadurch gekennzeichnet, dass** es folgende Schritte umfasst:
a) Mischen mindestens eines assoziativen Polymers aus:
- mindestens einem Monomer, bei dem es sich um (Meth)acrylsäure handelt,
- mindestens einem Monomer, bei dem es sich um einen (Meth)acrylsäureester handelt,
- und mindestens einem assoziativen hydrophoben Monomer mit mindestens einem Öl und Wasser in solchen Anteilen, dass das Öl mindestens 4 % des Gesamtgewichts der Mischung ausmacht,
b) Einstellen des pH-Werts der in Schritt a) erhaltenen Mischung auf einen Wert von mehr als 6, vorzugsweise 7, ganz besonders bevorzugt 8, und Erhalt einer wässrigen Emulsion,
c) Fällen der nach Schritt b) erhaltenen Mischung durch Einstellung des pH-Werts auf einen Wert von weniger als 6 zum Erhalt einer Dispersion von Teilchen aus dem Polymer und dem Öl in Wasser,
d) gegebenenfalls Isolieren der nach Schritt c) erhaltenen Teilchen aus dem Polymer und dem Öl durch Entfernung des Wassers.

2. Verfahren nach Anspruch 1, **dadurch gekennzeichnet, dass** dabei ausschließlich die Schritte a), b), c) und d) durchgeführt werden.

3. Verfahren nach einem der Ansprüche 1 bis 2, **dadurch gekennzeichnet, dass** in Schritt a) 0,1 bis 20 Trockengew.-%, vorzugsweise 0,1 bis 10 Trockengew.-% und besonders bevorzugt 0,1 bis 5 Trockengew.-% des assoziativen Polymers, bezogen auf das Gesamtgewicht der nach Schritt a) erhaltenen Mischung, verwendet werden.

4. Verfahren nach einem der Ansprüche 1 bis 3, **dadurch gekennzeichnet, dass** in Schritt a) mindestens 4 Gew.-%, vorzugsweise mindestens 10 Gew.-%, besonders bevorzugt mindestens 40 Gew.-% und ganz besonders bevorzugt mindestens 60 Gew.-% mindestens eines Öls und höchstens 70 Gew.-% mindestens eines Öls, bezogen auf das Gesamtgewicht der nach Schritt a) erhaltenen Mischung, verwendet werden.

5. Verfahren nach einem der Ansprüche 1 bis 4, **dadurch gekennzeichnet, dass** der pH-Wert der Mischung im Lauf von Schritt b) mit einer organischen oder anorganischen Base eingestellt wird.

6. Verfahren nach einem der Ansprüche 1 bis 5, **dadurch gekennzeichnet**, das im Lauf von Schritt c) eine mittelstarke oder starke Säure verwendet wird.

7. Verfahren nach einem der Ansprüche 1 bis 6, **dadurch gekennzeichnet, dass** das Monomer, bei dem es sich um einen (Meth)acrylsäureester handelt, vorzugsweise aus Ethylacrylat, Butylacrylat, Methylmethacrylat und Mischungen davon ausgewählt ist.

8. Verfahren nach einem der Ansprüche 1 bis 7, **dadurch gekennzeichnet, dass** das assoziative hydrophobe Monomer die allgemeine Formel (I) aufweist: 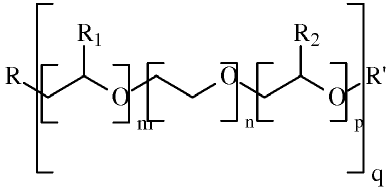 wobei:
- m, n, p und q ganze Zahlen sind und m, n und p kleiner als 150 sind, q größer als 0 ist und mindestens eine ganze Zahl von m, n und p nicht null ist,
- R eine polymerisierbare Vinylfunktion umfasst,
- R₁ und R₂ gleich oder verschieden sind und für Wasserstoffatome oder Alkylgruppen stehen,
- R' eine hydrophobe Gruppe mit mindestens 6 und höchstens 36 Kohlenstoffatomen, vorzugsweise mindestens 16 und höchstens 24 Kohlenstoffatomen und besonders bevorzugt mindestens 18 und höchstens 22 Kohlenstoffatomen ist.

9. Verfahren nach einem der Ansprüche 1 bis 8, **dadurch gekennzeichnet, dass** das Wasser im Lauf von Schritt d) durch Verdampfung oder Zentrifugation entfernt wird.

10. Verfahren nach einem der Ansprüche 1 bis 9, **dadurch gekennzeichnet, dass** das Öl aus Mikroalgenöl, Pongamia-pinnata-Öl, Jatrophaöl, Palmöl, Sonnenblumenöl, Rapsöl, Mandelöl, Erdnussöl, Kokosöl, Leinöl, Maisöl, Olivenöl, Traubenkernöl, Ricinusöl, Sesamöl, Senföl, Walsnussöl, Sojaöl, Walöl, Walratöl, Lebertran, Klauenöl, Rindertalg oder -fett, Schweineschmalz oder -fett, Borretschöl, Jojobaöl, Macadamiaöl, Johanniskrautöl, Haselnussöl, Moschusrosenöl, Aprikosenkernöl, Weizenkeimöl, Nachtkerzenöl, Entenfett, Hühnerfett, Ölsäure, Palmitinsäure, Linolsäure, Stearinsäure und Motorenölen ausgewählt wird.

11. Dispersion von Teilchen in Wasser, erhalten gemäß Schritt c) des Verfahrens nach einem der Ansprüche 1 und 3 bis 10, **dadurch gekennzeichnet, dass** die Teilchen mindestens ein Öl und mindestens ein assoziatives hydrophobes Polymer aus:
- mindestens einem Monomer, bei dem es sich um (Meth)acrylsäure handelt,
- mindestens einem Monomer, bei dem es sich um einen (Meth)acrylsäureester handelt,
- und mindestens einem assoziativen hydrophoben Monomer
enthalten und dass sie einen pH-Wert von weniger als 6 aufweist
und dass sie mindestens 4 Gew.-% Öl, bezogen auf ihr Gesamtgewicht, enthält.

12. Dispersion von Teilchen in Wasser nach Anspruch 11, **dadurch gekennzeichnet, dass** sie 0,1 bis 20 Trockengew.-%, vorzugsweise 0,1 bis 10 Trockengew.-% und besonders bevorzugt 0,1 bis 5 Trockengew.-% mindestens eines assoziativen hydrophoben Polymers, bezogen auf ihr Gesamtgewicht, enthält.

13. Dispersion von Teilchen in Wasser nach einem der Ansprüche 11 oder 12, **dadurch gekennzeichnet, dass** sie mindestens 4 Gew.-%, vorzugsweise mindestens 10 Gew.-%, besonders bevorzugt mindestens 40 Gew.-% und ganz besonders bevorzugt mindestens 60 Gew.-% mindestens eines Öls und höchstens 70 Gew.-% mindestens eines Öls, bezogen auf ihr Gesamtgewicht, enthält.

14. Dispersion von Teilchen in Wasser nach einem der Ansprüche 11 bis 13, **dadurch gekennzeichnet, dass** das Monomer, bei dem es sich um einen (Meth)acrylsäureester handelt, vorzugsweise aus Ethylacrylat, Butylacrylat, Methylmethacrylat und Mischungen davon ausgewählt ist.

15. Dispersion von Teilchen in Wasser nach einem der Ansprüche 11 bis 14, **dadurch gekennzeichnet, dass** das assoziative hydrophobe Monomer die allgemeine Formel (I) aufweist: 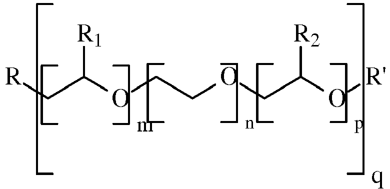 wobei:
- m, n, p und q ganze Zahlen sind und m, n und p kleiner als 150 sind, q größer als 0 ist und mindestens eine ganze Zahl von m, n und p nicht null ist,
- R eine polymerisierbare Vinylfunktion umfasst,
- R₁ und R₂ gleich oder verschieden sind und für Wasserstoffatome oder Alkylgruppen stehen,
- R' eine hydrophobe Gruppe mit mindestens 6 und höchstens 36 Kohlenstoffatomen, vorzugsweise mindestens 16 und höchstens 24 Kohlenstoffatomen und besonders bevorzugt mindestens 18 und höchstens 22 Kohlenstoffatomen ist.

16. Dispersion von Teilchen in Wasser nach einem der Ansprüche 11 bis 15, **dadurch gekennzeichnet, dass** das Öl aus Mikroalgenöl, Pongamia-pinnata-Öl, Jatrophaöl, Palmöl, Sonnenblumenöl, Rapsöl, Mandelöl, Erdnussöl, Kokosöl, Leinöl, Maisöl, Olivenöl, Traubenkernöl, Ricinusöl, Sesamöl, Senföl, Walsnussöl, Sojaöl, Walöl, Walratöl, Lebertran, Klauenöl, Rindertalg oder -fett, Schweineschmalz oder -fett, Borretschöl, Jojobaöl, Macadamiaöl, Johanniskrautöl, Haselnussöl, Moschusrosenöl, Aprikosenkernöl, Weizenkeimöl, Nachtkerzenöl, Entenfett, Hühnerfett, Ölsäure, Palmitinsäure, Linolsäure, Stearinsäure und Motorenölen ausgewählt ist.

17. Verwendung von wässrigen Dispersionen nach einem der Ansprüche 11 bis 16 auf den Gebieten Kosmetik, Anstrichmittel, Bauwesen, Brennstoffe, Schmiermittel, Antischaummittel, Metallurgie, Düngemittel, Pharmazie, Agrochemie, Pflanzenschutz, Detergentien, Lebensmittel, Leder und Beschichtung.

## Claims

1. Method for producing an aqueous formulation or optionally of solid particles containing at least one oil, and **characterized in that** it comprises the following steps:
a) mixing at least one associative polymer constituted of:
- at least one monomer which is (meth)acrylic acid,
- at least one monomer which is a (meth)acrylic ester,
- and at least one associative hydrophobic monomer,
with at least one oil and water, in proportions such that the oil represents at least 4% of the total weight of the mixture,
b) adjusting the pH of the mixture obtained in step a) to a value greater than 6, preferentially 7, very preferentially 8, and obtaining an aqueous emulsion.
c) precipitating the mixture obtained after step b) by adjusting the pH to a value less than 6, in view of obtaining a dispersion in water of the particles constituted of said polymer and of said oil,
d) optionally isolating the particles constituted of said polymer and of said oil obtained after step c) by removing water.

2. Method according to claim 1, **characterized in that** it exclusively implements the steps a), b), c), and d).

3. Method according to one of claims 1 to 2, **characterized in that**, during step a), 0.1% to 20%, preferentially 0.1% to 10%, very preferentially 0.1% to 5% by dry weight of said associative polymer, in relation to the total weight of the mixture obtained after step a), is implemented.

4. Method according to one of claims 1 to 3, **characterized in that** during step a), at least 4%, preferentially at least 10%, very preferentially at least 40%, extremely preferentially at least 60% by weight of at least one oil, and at most 70% by weight of at least one oil, compared to the total weight of the mixture obtained after step a), is implemented.

5. Method according to one of claim 1 to 4, **characterized in that** the pH of the mixture, during step b), is adjusted by means of an organic or mineral base.

6. Method according to one of claims 1 to 5, **characterized in that** a strong or a medium acid is implemented during step c).

7. Method according to one of claims 1 to 6, **characterized in that** the monomer, which is a (meth)acrylic ester is preferentially chosen from ethyl acrylate, butyl acrylate, methyl methacrylate, and mixtures thereof.

8. Method according to one of claims 1 to 7, **characterized in that** said associative hydrophobic monomer possesses the general formula (I): 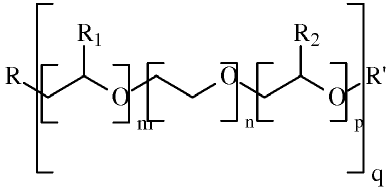 wherein:
- m, n, p and q are integers and m, n, p are less than 150, q is greater than 0, and at least one integer among m, n and p is non-zero;
- R comprises a polymerizable vinylic function,
- R₁ and R₂ are identical or different, and represent hydrogen atoms or alkyl groups,
- R' is a hydrophobic group comprising at least 6 and at most 36 carbon atoms, preferentially at least 16 and at most 24 carbon atoms, very preferentially at least 18 and at most 22 carbon atoms.

9. Method according to one of claims 1 to 8, **characterized in that** water is eliminated by evaporation or centrifugation during step d).

10. Method according to one of claims 1 to 9, **characterized in that** the oil is chosen from among microalgae oils, Pongamia pinnata oil, Jatropha oil, palm oil, sunflower oil, canola oil, almond oil, peanut oil, coconut oil, linseed oil, corn oil, olive oil, grapeseed oil, castor oil, sesame oil, mustard oil, walnut oil, soybean oil, whale oil, sperm oil, cod liver oil, neatsfoot oil, beef tallow or fat, pork fat or lard, borago oil, jojoba oil, macadamia oil, St. John's-wort oil, hazelnut oil, musk rose oil, apricot pit oil, wheatgerm oil, evening primrose oil, duck fat, chicken fat, oleic acid, palmitic acid, linoleic acid, stearic acid, and motor oils.

11. Dispersion of particles in water obtained after step c) of the process according to one of claims 1 and 3 to 10, **characterized in that** said particles contain at least one oil and at least one associative hydrophobic polymer constituted of :
- at least one monomer which is (meth)acrylic acid,
- at least one monomer which is a (meth)acrylic ester,
- and at least one associative hydrophobic monomer,
**in that** it possesses a pH less than 6,
and **in that** it contains at least 4% by weight of oil, in relation to its total weight.

12. Dispersion of particles in water according to claim 11, **characterized in that** it contains 0.1% to 20%, preferentially 0.1% to 10%, preferentially 0.1% to 5% by dry weight of at least an associative hydrophobic polymer, in relation to its total weight.

13. Dispersion of particles in water according to one of claims 11 or 12, **characterized in that** it contains at least 4%, preferentially at least 10%, very preferentially at least 40%, extremely preferentially at least 60% by weight of at least one oil, and at most 70% by weight of at least one oil, in relation to its total weight.

14. Dispersion of particles in water according to one of claims 11 to 13, **characterized in that** the monomer, which is a (meth)acrylic ester, is preferentially chosen from ethyl acrylate, butyl acrylate, methyl methacrylate, and mixtures thereof.

15. Dispersion of particles in water according to one of claims 11 to 14, **characterized in that** said associative hydrophobic monomer possesses the general formula (I): 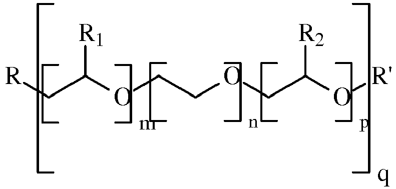 wherein:
- m, n, p and q are integers and m, n, p are less than 150, q is greater than 0, and at least one integer among m, n and p is non-zero
- R comprises a polymerizable vinylic function,
- R₁ and R₂ are identical or different, and represent hydrogen atoms or alkyl groups,
- R' is a hydrophobic group comprising at least 6 and at most 36 carbon atoms, preferentially at least 16 and at most 24 carbon atoms, very preferentially at least 18 and at most 22 carbon atoms.

16. Dispersion of particles in water according to one of claims 11 to 15, **characterized in that** the oil is chosen from among microalgae oils, Pongamia pinnata oil, Jatropha oil, palm oil, sunflower oil, canola oil, almond oil, peanut oil, coconut oil, linseed oil, corn oil, olive oil, grapeseed oil, castor oil, sesame oil, mustard oil, walnut oil, soybean oil, whale oil, sperm oil, cod liver oil, neatsfoot oil, beef tallow or fat, pork fat or lard, borago oil, jojoba oil, macadamia oil, St. John's-wort oil, hazelnut oil, musk rose oil, apricot pit oil, wheatgerm oil, evening primrose oil, duck fat, chicken fat, oleic acid, palmitic acid, linoleic acid, stearic acid, and motor oils.

17. Use of aqueous dispersions according to one of claims 11 to 16, in the fields of cosmetics, paints, construction, fuels, lubricants, anti-foaming agents, metallurgy, fertilizers, pharmaceuticals, agrochemistry, plant health, detergents, food, leather, surface application.
